# EUROPEAN PATENT APPLICATION

(11) **EP 4 349 849 A1**
(43) Date of publication of application: **10.04.2024**
(21) Application number: 22811306.4
(22) Date of filing: 24.05.2022
(51) Int. Cl.: C07K 1/34, B01D 61/14, B01D 61/58, B01D 63/02, B01D 69/00, B01D 69/02, B01D 69/08, C12M 1/12

(54) **METHOD AND APPARATUS FOR SEPARATING AND PURIFYING MINUTE USEFUL SUBSTANCE**

(30) Priority: 28.05.2021 JP 2021089878
(71) Applicant: Daicen Membrane-Systems Ltd., Tokyo 108-8230 (JP)
(72) Inventor: NAKATSUKA Shuji, Tokyo 108-8230 (JP)
(74) Representative: Grünecker Patent- und Rechtsanwälte PartG mbB
(86) International application number: PCT/JP2022/021194
(87) International publication number: WO 2022/250036

(57) **Abstract**

[Object] To provide a method for isolating and purifying a minute useful substance. [Solution] Disclosed is a method for isolating and purifying a minute useful substance. The method includes filtering a liquid containing a minute useful substance through a hollow fiber membrane. The hollow fiber membrane has an inner diameter of 0.2 mm to 1.4 mm and a molecular weight cut-off of 100000 to 1000000. The filtering includes a first filtration process of press-fitting the liquid containing the minute useful substance from a first opening on one end side of the hollow fiber membrane and filtering the liquid to separate the liquid into a permeate and a first concentrate, and a second filtration process of press-fitting the first concentrate from a second opening on the other end side of the hollow fiber membrane and filtering the first concentrate to separate the first concentrate into a permeate and a second concentrate. A concentrate is produced in which a concentration of the minute useful substance is increased by filtration in which the first filtration process and the second filtration process are alternately performed a plurality of times at a membrane surface velocity of 0.3 m/sec to 2 m/sec.

## Description

### Technical Field

The present disclosure relates to an isolation and purification method for isolating and purifying a minute useful substance selected from, for example, an exosome, an antibody, a virus, a protein, a nucleic acid, and the like, and an isolation and purification device for performing the isolation and purification method.

### Background Art

As a method for isolating and purifying a useful substance from a culture solution, a method using an isolation membrane is known.

JP H3-39084 discloses an invention of a method for concentrating a single cell alga culture solution, in which regular downflow washing is performed when the single cell alga culture solution is concentrated by being subjected to cross-flow filtration using a hollow-fiber ultrafiltration membrane (UF membrane) module having a molecular weight cut-off of 10000 to 1000000. When the culture solution is concentrated by cross-flow filtration, a concentrate is present outside the UF membrane and a permeate enters the inside of the UF membrane. When the downflow washing is performed, a cleaning water enters the inside of the UF membrane and then comes out of the UF membrane, thereby washing the UF membrane.

JP 2018-76291 describes an invention of a method for recovering useful substances. The method includes a bleeding process of discharging a culture solution from a cell bioreactor and adding a fresh culture medium in the same amount as the discharged culture solution to the bioreactor, and a filtration process of filtering the culture solution extracted from the bioreactor using a porous membrane having substantially no dense layer. The filtration in the filtration process is tangential flow filtration, and a velocity of a permeate in the filtration process is 1.0 LMH or less. It is described that the useful substance is selected from the group consisting of proteins, viruses, exosomes, and nucleic acids. It is described that alternating tangential flow filtration can also be carried out as the tangential flow filtration.

### Summary of Invention

An object of the present disclosure is to provide an isolation and purification method for isolating and purifying a minute useful substance selected from, for example, an exosome, an antibody, a virus, a protein, a nucleic acid, and the like. Another object of the present disclosure is to provide an isolation and purification device for carrying out the isolation and purification method.

According to one aspect of the present disclosure, a method for isolating and purifying a minute useful substance is provided. The method includes filtering a liquid containing a minute useful substance through a hollow fiber membrane.

The hollow fiber membrane has an inner diameter of 0.2 mm to 1.4 mm and a molecular weight cut-off of 100000 to 1000000. The filtering includes a first filtration process of press-fitting the liquid containing the minute useful substance from a first opening on one end side of the hollow fiber membrane and filtering the liquid to separate the liquid into a permeate and a first concentrate, and a second filtration process of press-fitting the first concentrate from a second opening on the other end side of the hollow fiber membrane and filtering the first concentrate to separate the first concentrate into a permeate and a second concentrate. A concentrate is produced in which a concentration of the minute useful substance is increased by alternating tangential flow filtration in which the first filtration process and the second filtration process are alternately performed a plurality of times. A membrane surface velocity in the first filtration process and the second filtration process is 0.3 m/sec to 2 m/sec. The membrane surface velocity is a linear velocity on a membrane surface.

According to one aspect of the present disclosure, in the filtering, the liquid containing the minute useful substance is filtered with a microfiltration membrane having a pore size of 0.1 µm to 0.5 µm, and then alternately performing the first filtration process and the second filtration process a plurality of times using a filtrate of the microfiltration membrane.

According to one aspect of the present disclosure, when the first filtration process is performed, the liquid containing the minute useful substance or the second concentrate produced in the second filtration process may be diluted by adding a buffer solution, and then the first filtration process may be performed.

According to one aspect of the present disclosure, in the first filtration process and the second filtration process, press-fitting may be performed by introducing a gas selected from the group consisting of nitrogen gas, inert gas, carbon dioxide, and air filtered by a HEPA filter.

According to one aspect of the present disclosure, the minute useful substance may be selected from the group consisting of an exosome, an antibody, a virus, a protein, and a nucleic acid.

According to one aspect of the present disclosure, the hollow fiber membrane may be a hollow fiber membrane module in which a plurality of hollow fiber membranes is accommodated in a case housing having a plurality of liquid inlet/outlet ports.

According to one aspect of the present disclosure, when the first filtration process and the second filtration process are alternately performed, a dilution factor of an object to be filtered in the first filtration process may be increased as the number of times of performing the first filtration process and the second filtration process increases.

According to one aspect of the present disclosure, when the first filtration process and the second filtration process are alternately performed, a dilution factor of an object to be filtered in the first filtration process may be increased in a range of 2 times in volume to 15 times in volume as the number of times of performing the first filtration process and the second filtration process increases.

According to another aspect of the present disclosure, an isolation and purification device for isolating and purifying a minute useful substance is provided. The isolation and purification device includes: a first tank into which a liquid containing the minute useful substance enters; a second tank disposed at a distance from the first tank; a hollow fiber membrane disposed to connect a fluid inlet/outlet port of the first tank and a fluid inlet/outlet port of the second tank; a buffer solution tank connected to the first tank by a solution feed line to perform liquid feeding;
a permeate tank that stores a permeate filtered by the hollow fiber membrane; and a pressurizing device configured to pressurize one of the liquid in the first tank and a liquid in the second tank. The isolation and purification device may be used to carry out the method for isolating and purifying the minute useful substance of the present disclosure.

According to still another aspect of the present disclosure, an isolation and purification device for isolating and purifying a minute useful substance is provided, The isolation and purification device includes: a microfiltration membrane having a pore size of 0.1 µm to 0.5 µm;
a first tank which is connected to a filtrate outlet of the microfiltration membrane by a solution feed line and into which a liquid containing the minute useful substance enters;
a second tank disposed at a distance from the first tank; a hollow fiber membrane disposed to connect a fluid inlet/outlet port of the first tank and a fluid inlet/outlet port of the second tank; a buffer solution tank connected to the first tank by the solution feed line to be able to perform liquid feeding; a permeate tank that stores a permeate filtered by the hollow fiber membrane; and a pressurizing device configured to pressurize one of the liquid in the first tank and a liquid in the second tank. The isolation and purification device may be used to carry out the method for isolating and purifying the minute useful substance of the present disclosure.

According to one aspect of the present disclosure, the first tank and the second tank may be transparent and thus a liquid level therein can be visually observed.

According to one aspect of the present disclosure, a portion including the fluid inlet/outlet port of the first tank has a connection portion with the hollow fiber membrane, and the connection portion may have a conical inclined surface with a diameter decreasing from a side of the first tank to a side of the hollow fiber membrane side, and a portion including the fluid inlet/outlet port of the second tank has a connection portion with the hollow fiber membrane, and the connection portion may have a conical inclined surface with a diameter decreasing from a side of the second tank to a side of the hollow fiber membrane.

According to one aspect of the present disclosure, the hollow fiber membrane may be a hollow fiber membrane module in which a hollow fiber membrane bundle is accommodated in a case housing. The hollow fiber membrane bundle may have at least one end side sealed with an adhesive and which includes 5 to 50 hollow fiber membranes. The case housing may have three liquid inlet/outlet ports in three directions. One of the three liquid inlet/outlet ports may be a permeate port. The permeate port may be connected to a permeate tank, and the other two of the three liquid inlet/outlet ports may be connected to a fluid inlet/outlet port of the first tank and a fluid inlet/outlet port of the second tank, respectively.

According to the method and device for isolating and purifying the minute useful substance of the present disclosure, a minute useful substance such as exosomes, antibodies, viruses, proteins, and nucleic acids can be concentrated to a high concentration.

### Brief Description of Drawings

FIG. 1 is a diagram illustrating an isolation and purification flow using an isolation and purification device that can carry out a method for isolating and purifying a minute useful substance.
FIG. 2 is a partially enlarged view of an isolation and purification device of an embodiment different from that of FIG. 1, which is used in an isolation and purification flow shown in FIG. 1.

### Description of Embodiments

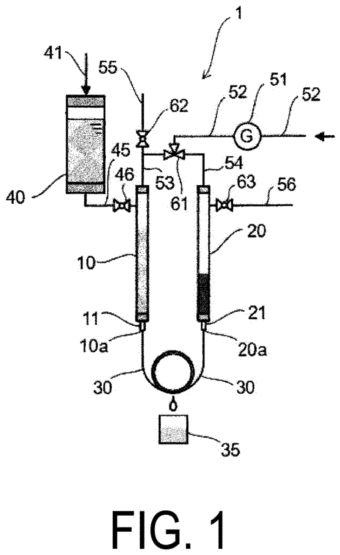

One embodiment of a method for isolating and purifying a minute useful substance will be described with reference to a production flow using an isolation and purification device 1 shown in FIG. 1.

In a first process, a liquid containing a minute useful substance is put into a first tank 10. When the liquid containing the minute useful substance is a culture solution containing the minute useful substance, it is preferable to put a supernatant into the first tank 10. Examples of the minute useful substance include substances selected from exosomes, antibodies, viruses, proteins, nucleic acids, and the like.

Although the first tank 10 has a cylindrical shape in FIG. 1, the shape is not limited thereto, and the shape and the volume can be determined according to the situation of the installation site and the amount of treatment.

The first tank 10 is preferably transparent such that a liquid level therein can be visually observed, and is preferably made of a water-repellent material to prevent a liquid from adhering to and remaining on an inner wall surface of the first tank 10.

It is preferable that a part or the whole of the first tank 10 is made of, for example, polycarbonate, fluororesin, or an acrylic resin such as polyacrylonitrile or polyacrylate.

The first tank 10 may be provided with a withdrawal line for withdrawing a final concentrate from the first tank 10.

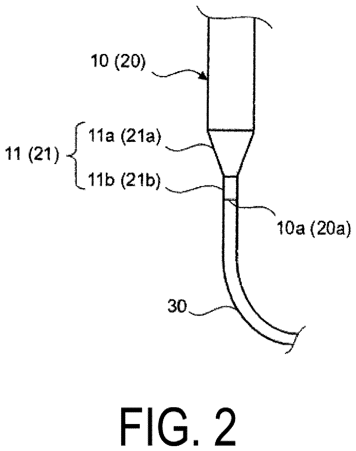

As exemplarily shown in FIG. 2, a connecting portion 11 with a hollow fiber membrane 30 of a portion including a fluid inlet/outlet port 10a of the first tank 10 may have a conical inclined surface 11a and a cylindrical vertical surface 11b such that the diameter decreases from the first tank 10 side to the hollow fiber membrane 30 side.

Although the connecting portion 11 has the conical inclined surface 11a and the cylindrical vertical surface 11b in FIG. 2, the cylindrical vertical surface 11b may be omitted as long as the connecting portion 11 has the conical inclined surface 11a.

When the connecting portion 11 shown in FIG. 2 is provided, a liquid containing the minute useful substance or a concentrate thereof can be prevented from staying on a bottom side of the first tank 10, and the recovery rate of the minute useful substance can be increased, which is preferable.

Before the first process, a pretreatment process using a microfiltration membrane (microfiltration membrane module) can be performed as necessary. The microfiltration membrane (microfiltration membrane module) preferably has a pore size of 0.1 µm to 0.5 µm.

In the pretreatment process, a filtrate (pretreatment liquid) produced by filtering the liquid containing the minute useful substance with a microfiltration membrane (microfiltration membrane module) can be sent to the first tank 10.

In the second process, the liquid containing the minute useful substance (or pretreatment liquid) in the first tank 10 can be diluted by supplying a buffer solution in a buffer solution tank 40 from a buffer solution feed line 45 into the first tank 10 in a state where an opening/closing valve (electromagnetic valve or the like) 46 is opened.

In a state where the first tank 10 contains a diluent of the liquid containing the minute useful substance (or pretreatment liquid), a space in which the diluent is not present remains in an upper portion of the first tank 10.

As the buffer solution in the buffer solution tank 40, a medical or biochemical buffer solution is preferable, and for example, a phosphate buffer solution (PBS), a tris-hydrochloric acid buffer solution, a sodium citrate buffer solution, a citrate phosphate buffer solution, an acetate buffer solution, a borate buffer solution, or the like can be used.

When the buffer solution is replenished to the buffer solution tank 40, the buffer solution is replenished from a buffer solution replenishing line 41.

The order of the first process and the second process may be reversed so that the buffer solution in the buffer solution tank 40 is supplied from the buffer solution feed line 45 into the first tank 10, and then the liquid containing the minute useful substance is put in the first tank 10.

Alternatively, the first process and the second process may be combined into one process, and the liquid containing the minute useful substance and the buffer solution may be added and mixed in a separately provided mixing tank to prepare a diluent of the liquid containing the minute useful substance, and then the diluent may be put in the first tank 10.

In the third process, a first filtration process is performed in which a pump (not shown) or the like is operated to supply gas from a gas supply source (not shown) into the first tank 10 to pressurize the liquid containing the minute useful substance in the first tank 10, thereby press-fitting the liquid containing the minute useful substance into the hollow fiber membranes 30 and filtering the liquid.

In the present disclosure, all processes of filtering the liquid in the first tank 10 with the hollow fiber membrane 30 and sending the liquid to the second tank 20 are referred to as the first filtration process.

The gas for pressurization is sent through a gas supply line 52 provided with a pressure gauge 51 and a first tank gas supply line 53 by switching a three-way valve 61. At this time, an opening/closing valve 46 and an opening/closing valve 62 of a first gas vent line 55 are closed, and an opening/closing valve 63 of a second gas vent line 56 is opened.

As the gas for pressurization, it is possible to use a gas selected from inert gases such as nitrogen gas, argon, and helium, carbon dioxide, and clean air filtered by a HEPA filter or the like.

The filtered permeate is stored in a permeate tank 35, and the concentrate (first concentrate) containing the minute useful substance is sent to the second tank 20.

In a state where the second tank 20 contains the first concentrate, a space in which the first concentrate is not present remains in an upper portion of the second tank 20.

An inner diameter of the hollow fiber membrane 30 is preferably 0.2 mm to 1.4 mm, more preferably 0.2 mm to 1.0 mm, and still more preferably 0.4 mm to 1.0 mm.

As the hollow fiber membrane 30, an ultrafiltration membrane having a molecular weight cut-off of 100000 to 1000000 is preferable, an ultrafiltration membrane having a molecular weight cut-off of 200000 to 800000 is more preferable, and an ultrafiltration membrane having a molecular weight cut-off of 300000 to 600000 is still more preferable. The molecular weight cut-off may be evaluated by permeability % of γ-globulin in a phosphate buffer solution ((γ-globulin concentration in permeate/γ-globulin concentration in solution (100 mg/L)) × 100) when a solution of 100 mg/L of γ-globulin (from Sigma-Aldrich, bovine serum γ-globulin, molecular weight: 150000) in a phosphate buffer solution is subjected to cross-flow filtration (membrane surface velocity: 0.2 m/s) through the hollow fiber membrane 30 at a filtration pressure of 0.1 MPa. The hollow fiber membrane 30 has a permeability of y-globulin of preferably 5% to 95%, more preferably 10% to 80%, and still more preferably 30% to 70%.

The hollow fiber membrane 30 may be a hydrophobic membrane such as a polyethersulfone membrane or may be a cellulose-based hydrophilic membrane, and is preferably the cellulose-based hydrophilic membrane. Examples of the cellulose-based hydrophilic membrane include a cellulose acetate membrane, a regenerated cellulose membrane, a cellulose propionate membrane, a cellulose butyrate membrane, and a cellulose benzoate membrane.

As the hollow fiber membrane 30, FUS5082 (polyethersulfone membrane; molecular weight cut-off: 500000, γ-globulin permeability: 70%) from Daicen Membrane-Systems Ltd., FUC1582 (cellulose acetate membrane; molecular weight cut-off: 150000, γ-globulin permeability: 10%) from Daicen Membrane-Systems Ltd., or the like can be used.

In the example shown in FIG. 1, the hollow fiber membrane 30 is disposed to connect the fluid inlet/outlet port 10a of the first tank 10 and the fluid inlet/outlet port 20a of a connecting portion 21 of the second tank 20.

The hollow fiber membrane 30 and the fluid inlet/outlet port 10a of the first tank 10 may be connected, for example, by fitting an opening end portion of the hollow fiber membrane 30 into a narrow tube such as a syringe needle fixed to the fluid inlet/outlet port 10a side of the first tank 10. The hollow fiber membrane 30 and the fluid inlet/outlet port 20a of the second tank 20 can be connected in the same manner.

The permeate tank 35 is for storing the permeate produced by filtration in the hollow fiber membrane 30. Although the permeate tank 35 is shown as being small in FIG. 1, the permeate tank 35 may be so large a tank as to contain most of the hollow fiber membrane 30.

Although one hollow fiber membrane 30 is shown in FIG. 1, a plurality of the hollow fiber membranes 30 may be used, and for example, a hollow fiber membrane bundle of 2 to 150 hollow fiber membranes may be used.

A hollow fiber membrane module in which the plurality of hollow fiber membranes (hollow fiber membrane bundle) 30 is accommodated in a case housing having a plurality of liquid inlet/outlet ports may be used. When the hollow fiber membrane bundle is used, one end or both ends may be integrated with an adhesive.

When the hollow fiber membrane module is used, a plurality of liquid inlet/outlet ports of the hollow fiber membrane module, the fluid inlet/outlet port 10a of the first tank 10, and the fluid inlet/outlet port 20a of the second tank 20 may be connected, and a liquid inlet/outlet port (liquid permeation outlet port) of the rest of the hollow fiber membrane module and the permeate tank 35 may be connected.

It is preferable that the filtration in the third process is performed at a membrane surface velocity in a range of 0.3 m/sec to 2 m/sec, and it is more preferable that the filtration is performed at a membrane surface velocity in a range of 0.5 m/sec to 1.5 m/sec. When the membrane surface velocity is lower than 0.3 m/sec, purification efficiency is reduced. On the contrary, when the membrane surface velocity is higher than 2 m/sec, a pressure level to increase the membrane surface velocity becomes too high, and a shearing force applied to the minute useful substance during filtration also becomes too high, which may result in denaturation of the minute useful substance.

In a method for maintaining the membrane surface velocity within the above range, a pressure of an inlet pressure of the hollow fiber membrane 30 (the fluid inlet/outlet port 10a side of the first tank 10) is preferably adjusted to 0.01 MPa to 0.2 MPa, more preferably 0.02 MPa to 0.15 MPa, and still more preferably 0.03 MPa to 0.12 MPa.

In the method for maintaining the membrane surface velocity within the above range, a pressure of an outlet pressure of the hollow fiber membrane 30 (the fluid inlet/outlet port 20a side of the second tank 20) is preferably adjusted to 0.03 MPa or less, more preferably 0.01 MPa or less, and still more preferably 0 MPa.

In the fourth process, a second filtration process is performed in which a pump (not shown) or the like is operated to supply gas from a gas supply source (not shown) into the second tank 20 to pressurize the liquid (first concentrate) containing the minute useful substance in the second tank 20, and the liquid containing the minute useful substance is filtered through the inside of the hollow fiber membrane 30.

In the present disclosure, all processes of filtering the liquid in the second tank 20 with the hollow fiber membrane 30 and sending the liquid to the first tank 10 are referred to as the second filtration process.

The filtered permeate is stored in the permeate tank 35, and the concentrate (second concentrate) containing the minute useful substance is sent to the first tank 10.

Although the second tank 20 has a cylindrical shape in FIG. 1, the shape is not limited thereto, and the shape and the volume can be determined according to the situation of the installation site and the amount of treatment.

The second tank 20 is preferably transparent such that the liquid level therein can be visually observed, and preferably has water repellency to prevent a liquid from adhering to and remaining on an inner wall surface of the second tank 20.

It is preferable that a part or the whole of the second tank 20 is made of, for example, polycarbonate, fluororesin, or an acrylic resin such as polyacrylonitrile or polyacrylate.

It is preferable that the first tank 10 and the second tank 20 have the same shape and the same volume. The first tank 10 and the second tank 20 may be arranged at the same height position with an interval therebetween.

The gas for pressurization is sent through the gas supply line 52 and a second tank gas supply line 54 by switching the three-way valve 61. At this time, an opening/closing valve 63 of a second gas vent line 56 and the opening/closing valve 46 are closed, and the opening/closing valve 62 of the first gas vent line 55 is opened.

As the gas for pressurization, it is possible to use a gas selected from inert gases such as nitrogen gas, argon, and helium, carbon dioxide, and clean air filtered by a HEPA filter or the like.

The membrane surface velocity in the fourth process is preferably in the same range as the membrane surface velocity in the third process.

To maintain the membrane surface velocity within the above range, the inlet pressure of the hollow fiber membrane 30 (the fluid inlet/outlet port 20a side of the second tank 20) in the fourth process is preferably adjusted to 0.01 MPa to 0.2 MPa, more preferably 0.02 MPa to 0.15 MPa, and still more preferably 0.03 MPa to 0.12 MPa.

To maintain the membrane surface velocity within the above range, the pressure of the outlet pressure of the hollow fiber membrane 30 (the fluid inlet/outlet port 10a side of the first tank 10) in the fourth process is preferably adjusted to 0.03 MPa or less, more preferably 0.01 MPa or less, and still more preferably 0 MPa.

The third process and the fourth process can be continuously performed by switching the three-way valve 61 while the gas is continuously supplied from the gas supply source through the gas supply line 52.

Thereafter, the minute useful substance in the liquid containing the minute useful substance may be isolated and purified by repeating the first filtration process (third process) and the second filtration process (fourth process) a plurality of times.

When the first filtration process and the second filtration process are repeated a plurality of times, it is preferable that a dilution factor with the buffer solution to be filtered in the first tank 10 in the first filtration process is increased as the number of repetitions increases, and, for example, the dilution factor can be increased in a range of 2 times in volume to 15 times in volume, and preferably in a range of 2 times in volume to 10 times by volume.

As described above, by alternating tangential flow filtration in which the first filtration process and the second filtration process are alternately performed, it is possible to produce a concentrate in which a concentration of the minute useful substance is increased.

Each aspect disclosed in the present specification can be combined with any other feature disclosed herein.

Note that the configurations, combinations thereof, and the like in each embodiment of the present disclosure are examples, and various configurational additions, omissions, substitutions, and other changes may be made, as appropriate, without departing from the spirit of the disclosure of the present invention. The present disclosure is not limited by the embodiment and is limited only by the claims.

### Examples

### Example 1

The isolation and purification device 1 shown in FIG. 1 was used according to the following specifications.
· First tank 10 and second tank 20
   Material: polyacrylonitrile
   Size: length 25 cm, inner diameter 0.25 cm, volume 120 cm³
· Buffer solution tank 40
   Volume: 1.6 L
· Hollow fiber membrane 30

Hollow fiber membrane (product name FUS5081, manufactured by Daicen Membrane-Systems Ltd.) made of polyethersulfone (PES) having an inner diameter of 0.8 mm, an outer diameter of 1.3 mm, a length of 50 cm, a membrane area of 12.6 cm², and a molecular weight cut-off of 500000.

### Preparation of Medium for Exosome

A basal medium and a supplement of Ultra ExoM (trade name) Culture Medium for Extracellular Vesicles (EVs) (manufactured by Santeja) were mixed at a mixing ratio of 9: 1 (volume ratio) to prepare 50 mL of a medium for exosome.

When proteins contained in the exosome medium were analyzed by SDS-PAGE (CBB staining), proteins having a molecular weight of about 70000 were mainly contained. As a result of quantitative analysis by a Bradford method, a total amount of protein in the medium was 107 mg. When insulin contained in the exosome medium was quantified by an (ELISA) method, the result was 3531 µg.

### Preparation of Liquid Sample Containing Exosome

Exosome HansaBioMed (Human plasma of health donors 100 µg [number of particles > 1 × 10¹⁰)]) manufactured by LONZA KK.) was added to 50 mL of the exosome medium prepared above to prepare 50 mL of a liquid sample containing the minute useful substance (exosome).

### Implementation of Method for Isolating and Purifying Exosome from Liquid Sample Containing Minute Useful Substance (Exosome)

The exosome was isolated and purified from the liquid sample containing the minute useful substance (exosome) by using the isolation and purification device shown in FIG. 1 showing the above specification. The isolation and purification were performed at room temperature (about 20°C).

(1) The entire amount of the liquid sample was filtered through an MF membrane having a pore size of 0.22 µm (Millex-GP, material: PES, manufactured by Nihon Millipore K.K.), which was set in a syringe cylinder, to produce a filtrate (pretreatment liquid).
(2) In a mixing vessel not shown in FIG. 1, 25 mL of the filtrate and 25 mL of phosphate buffer solution (PBS) were mixed to prepare 50 mL of liquid sample diluent.
(3) 50 mL of liquid sample diluent in the mixing vessel was put in the first tank 10.
(4) Nitrogen gas was supplied to an upper space in the first tank 10 at a pressure of 0. 1 MPa, and tangential flow filtration was performed while the liquid sample diluent was allowed to pass through the inside of the hollow fiber membrane 30.

At this time, the second tank 20 was opened to the atmosphere by opening an opening/closing valve 56, and the pressure was 0. The membrane surface linear velocity of the liquid sample diluent flowing inside the hollow fiber membrane 30, that is, the membrane surface velocity was 1.0 m/s. The membrane surface linear velocity was calculated from an increase rate of a concentrate amount in the second tank 20.

The permeate was stored in the permeate tank 35, and the concentrate (first concentrate) was transferred into the second tank 20 (first filtration process).

(5) When the liquid sample diluent in the first tank 10 passed through inside the hollow fiber membrane 30 and was filtered and most of the diluent was transferred to the second tank 20, the nitrogen gas was supplied to the second tank 20 by switching the three-way valve 61, and at the same time, the pressure in the first tank 10 was released by opening the opening/closing valve 62.
(6) By this operation, filtration was performed while transferring the first concentrate from the second tank 20 to the first tank 10, the permeate was stored in the permeate tank 35, and the concentrate (second concentrate) was transferred into the first tank 10 (second filtration process).

When most of the first concentrate in the second tank 20 transferred to the first tank 10, the second concentrate in the first tank 10 was filtered again by the hollow fiber membrane 30 by switching the three-way valve 61, and the concentrate was transferred into the second tank 20 (first filtration process).

The alternating tangential flow filtration in which the same first filtration process and the same second filtration process were repeated a plurality of times was performed.

While the first filtration process and the second filtration process (alternating tangential flow filtration) of (4) to (6) were repeated, when the amount of the concentrate in the first tank 10 reached about half (about 25 mL), 50 mL of phosphate buffer solution (calcium, magnesium-free phosphate buffered saline) (10 × PBS Buffer manufactured by Nippon Gene Co., Ltd.) in the buffer solution tank 40 was added to the first tank 10.

Nitrogen gas corresponding to a decrease in the buffer solution was supplied and sealed in a gas phase portion (space portion not containing the buffer solution) in the buffer solution tank 40.

The isolation and purification processes (alternating tangential flow filtration) of (4) to (6) were repeated 9 times in total. As a result, 450 mL of phosphate buffer solution in total was added to 50 mL of the initial liquid sample.

After the ninth addition of the phosphate buffer solution (concentrate amount: 75 mL), filtration was performed by the alternating tangential flow filtration without dilution until the amount reached 6 mL, thereby producing a concentrate having an increased exosome concentration.

When the surface of the hollow fiber membrane after isolation and purification was observed with an electron microscope (10000 times), an exosome having a size of about 100 nm was observed. It was conceivable that membrane permeation of the exosome was blocked by filtration using a hollow fiber membrane having a molecular weight cut-off of 500000 (equivalent membrane pore size of 20 nm) and it was determined that the exosome was sufficiently recovered by the membrane.

On the other hand, the total amount of protein and the amount of insulin in 6 mL of a final sample concentrate were 5.7 mg and 20 µg, respectively, and the total amount of protein and the amount of insulin could be reduced to 5.3% and 0.6%, respectively, with respect to 50 mL (total amount of protein: 107 mg, amount of insulin: 3531 µg) of the liquid sample containing the exosome.

In the alternating tangential flow filtration process described above, the amount of the filtrate was sampled over time, and a filtration rate was calculated from the mass change. Although a conversion filtration rate per 1 hour, membrane area of 1 m², and pressure of 0.1 MPa remarkably decreased at the initial stage, the filtration rate became substantially constant at 200 to 230 (average: 210) L/m² h from about 25 minutes after the start of filtration, and the isolation and purification were completed after about 88 minutes from the start of filtration.

### Comparative Example 1

The isolation and purification of the exosome were performed in the same manner as in Example 1 except that the length of the hollow fiber membrane in Example 1 was changed from 50 cm to 10 cm and no dilution with a phosphate buffer solution was performed. The filtration rate converted based on 1 hour, membrane area of 1 m², and pressure of 0.1 MPa was 275 L/m² h or more in the initial measurement value, but gradually decreased to less than 25 L/m² h after more than 200 minutes from the start of filtration, and the isolation and purification operation was stopped.

### Example 2

Using a hollow fiber membrane made of cellulose acetate (CA) (product name: FUC 1582, manufactured by Daicen Membrane-Systems Ltd.) having an inner diameter of 0.8 mm, an outer diameter of 1.3 mm, and a molecular weight cut-off of 150000 instead of the hollow fiber membrane of Example 1, the exosome was isolated and purified by the same device as in Example 1. At that time, 27 mL of the phosphate buffer solution was added to 3 mL of the liquid sample containing the exosome to produce 30 mL of liquid sample diluent.

The filtration rate converted based on 1 hour, membrane area of 1 m², and pressure of 0.1 MPa did not significantly decrease from 500 L/m² h in the initial measurement value, and was stable at about 420 L/m² h, and the filtration rate was about 2 times as high as that in Example 1, and thus the exosome was efficiently recovered. On the other hand, although the protein content in the permeate was smaller than that in Example 1, the total amount of protein in 0.1 mL of the final sample concentrate was 0.25 mg, and the total amount of protein could be reduced to 6.6% with respect to 3 mL (the total amount of protein was 3.8 mg) of the liquid sample containing the exosome.

### Comparative Example 2

The isolation and purification of the exosome were performed in the same manner as in Example 2 except that no buffer solution was added in the operation of Example 2. The filtration rate converted based on 1 hour, membrane area of 1 m², and pressure of 0.1 MPa greatly decreased from the initial value of 550 L/m² h, and remarkably decreased to about 200 L/m² h at 6 minutes after the start of filtration.

### Industrial Applicability

The isolation and purification method of the present disclosure can be used when a substance selected from an exosome, an antibody, a virus, a protein, a nucleic acid, and the like is isolated from a culture solution and purified.

### Reference Signs List

1 Isolation and purification device
10 First tank
20 Second tank
30 Hollow fiber membrane
35 Permeate tank
40 Buffer solution tank

## Claims

1. A method for isolating and purifying a minute useful substance, the method comprising filtering a liquid containing a minute useful substance through a hollow fiber membrane,
wherein the hollow fiber membrane has an inner diameter of 0.2 mm to 1.4 mm and a molecular weight cut-off of 100000 to 1000000,
the filtering includes
a first filtration process of press-fitting the liquid containing the minute useful substance from a first opening on one end side of the hollow fiber membrane and filtering the liquid to separate the liquid into a permeate and a first concentrate, and
a second filtration process of press-fitting the first concentrate from a second opening on the other end side of the hollow fiber membrane and filtering the first concentrate to separate the first concentrate into a permeate and a second concentrate,
a concentrate is produced in which a concentration of the minute useful substance is increased by alternating tangential flow filtration in which the first filtration process and the second filtration process are alternately performed a plurality of times, and
a membrane surface velocity in the first filtration process and the second filtration process is 0.3 m/sec to 2 m/sec.

2. The method for isolating and purifying a minute useful substance according to claim 1, wherein in the filtering, the liquid containing the minute useful substance is filtered with a microfiltration membrane having a pore size of 0.1 µm to 0.5 µm, and then the first filtration process and the second filtration process are alternately performed a plurality of times using a filtrate of the microfiltration membrane.

3. The method for isolating and purifying a minute useful substance according to claim 1 or 2, wherein when the first filtration process is performed, the liquid containing the minute useful substance or the second concentrate produced in the second filtration process is diluted by adding a buffer solution, and then the first filtration process is performed.

4. The method for isolating and purifying a minute useful substance according to any one of claims 1 to 3, wherein in the first filtration process and the second filtration process, press-fitting is performed by introducing a gas selected from the group consisting of nitrogen gas, inert gas, carbon dioxide, and air filtered by a HEPA filter.

5. The method for isolating and purifying a minute useful substance according to any one of claims 1 to 3, wherein the minute useful substance is selected from the group consisting of an exosome, an antibody, a virus, a protein, and a nucleic acid.

6. The method for isolating and purifying a minute useful substance according to any one of claims 1 to 3, wherein the hollow fiber membrane is a hollow fiber membrane module in which a plurality of hollow fiber membranes is accommodated in a case housing having a plurality of liquid inlet/outlet ports.

7. The method for isolating and purifying a minute useful substance according to any one of claims 1 to 6, wherein when the first filtration process and the second filtration process are alternately performed, a dilution factor of an object to be filtered in the first filtration process is increased as the number of times of performing the first filtration process and the second filtration process increases.

8. The method for isolating and purifying a minute useful substance according to any one of claims 1 to 6, wherein when the first filtration process and the second filtration process are alternately performed, a dilution factor of an object to be filtered in the first filtration process is increased in a range of 2 times in volume to 15 times in volume as the number of times of performing the first filtration process and the second filtration process increases.

9. An isolation and purification device for carrying out the method for isolating and purifying a minute useful substance described in claim 1, the isolation and purification device comprising:
a first tank into which a liquid containing the minute useful substance enters;
a second tank disposed at a distance from the first tank;
a hollow fiber membrane disposed to connect a fluid inlet/outlet port of the first tank and a fluid inlet/outlet port of the second tank;
a buffer solution tank connected to the first tank by a solution feed line to perform liquid feeding;
a permeate tank that stores a permeate filtered by the hollow fiber membrane; and
a pressurizing device configured to pressurize one of the liquid in the first tank and a liquid in the second tank.

10. An isolation and purification device for carrying out the method for isolating and purifying a minute useful substance described in claim 2, the isolation and purification device comprising:
a microfiltration membrane having a pore size of 0.1 µm to 0.5 µm;
a first tank which is connected to a filtrate outlet of the microfiltration membrane by a solution feed line and into which a liquid containing the minute useful substance enters;
a second tank disposed at a distance from the first tank;
a hollow fiber membrane disposed to connect a fluid inlet/outlet port of the first tank and a fluid inlet/outlet port of the second tank;
a buffer solution tank connected to the first tank by the solution feed line to perform liquid feeding;
a permeate tank that stores a permeate filtered by the hollow fiber membrane; and
a pressurizing device configured to pressurize one of the liquid in the first tank and a liquid in the second tank.

11. The isolation and purification device for carrying out the method for isolating and purifying a minute useful substance according to claim 9 or 10, wherein the first tank and the second tank is transparent and thus a liquid level therein can be visually observed.

12. The isolation and purification device for carrying out the method for isolating and purifying a minute useful substance according to claim 9 or 10, wherein
a portion including the fluid inlet/outlet port of the first tank has a connection portion with the hollow fiber membrane, and the connection portion has a conical inclined surface with a diameter decreasing from a side of the first tank to a side of the hollow fiber membrane, and
a portion including the fluid inlet/outlet port of the second tank has a connection portion with the hollow fiber membrane, and the connection portion has a conical inclined surface with a diameter decreasing from a side of the second tank to a side of the hollow fiber membrane.

13. The isolation and purification device for carrying out the method for isolating and purifying a minute useful substance according to any one of claims 9 to 12, wherein the hollow fiber membrane is a hollow fiber membrane module in which a hollow fiber membrane bundle is accommodated in a case housing, the hollow fiber membrane bundle having at least one end side sealed with an adhesive and including 5 to 50 hollow fiber membranes, the case housing has three liquid inlet/outlet ports in three directions, one of the three liquid inlet/outlet ports is a permeate port, the permeate port is connected to a permeate tank, and the other two of the three inlet/outlet ports are connected to a fluid inlet/outlet port of the first tank and a fluid inlet/outlet port of the second tank, respectively.
